Europäisches Patentamt

European Patent Office

Office européen des brevets

(11) Publication number: **0 417 823 A2**

# EUROPEAN PATENT APPLICATION

(21) Application number: 90202239.1

(51) Int. Cl.5: **C11C 3/00**, C12P 7/64

(22) Date of filing: 21.08.90

(30) Priority: 13.09.89 GB 8920715

(43) Date of publication of application:
20.03.91 Bulletin 91/12

(84) Designated Contracting States:
AT BE CH DE DK ES FR GB GR IT LI NL SE

(71) Applicant: UNILEVER NV
Burgemeester s'Jacobplein 1 P.O. Box 760
NL-3000 DK Rotterdam(NL)

(84) BE CH DE DK ES FR GR IT LI NL SE AT

Applicant: UNILEVER PLC
Unilever House Blackfriars
London EC4P 4BQ(GB)

(84) GB

(72) Inventor: Macrae, Alasdair Robin
Ivy Cottage, Newton Blossomville
Bedford MK43 8AN(GB)
Inventor: Padley, Frederick Bolton
25 Colchester Way, Bedford
Bedfordshire MK41 8BG(GB)
Inventor: Chandler, Ian Christopher
15 Northcote
Bedford MK41 8EX(GB)

(74) Representative: Kan, Jacob Hendrik, Dr. et al
Unilever N.V. Patent Division P.O. Box 137
NL-3130 AC Vlaardingen(NL)

(54) Transesterification.

(57) A process is described by which triglycerides-containing, short-chain fatty acid residues are interesterified enzymatically with long-chain fatty acids or their esters, while removing the liberated short-chain fatty acid compounds from the reaction mixture.

## TRANSESTERIFICATION

This invention relates to the transesterification of triglyceride esters of fatty acids under the influence as transesterification catalyst of lipase enzymes. The invention also relates to novel triglyceride esters prepared by such methods.

Under the influence of lipase enzyme as catalyst, triglyceride esters of fatty acids such as occur as the principal components of fats and glyceride oils undergo rearrangement of their fatty acid residues with those of an added source, for example, of free fatty acid or alkyl ester thereof. This has been disclosed in, e.g., GB 1,577,933, but also in GB 2,035,359. By this unique method, which is conducted in organic liquid phase comprising either a hydrocarbon compound or an ester of a long fatty acid, a cheap conversion of fats and glyceride oils into more valuable homologues with prized physical and/or therapeutic properties is provided. For example, a cheap oil or fat may be converted by the introduction of a physiologically significant fatty acid, such as eicosapentaenoic acid, into a pharmacologically valuable product. Similarly, by the use of an enzyme selectively active in 1-, 3-glyceride positions, a cheap oil may be converted into a fat with closely similar symmetrical disaturated triglycerides into those of cocoa butter and other hard vegetable butters, by the introduction in these positions of palmitic and/or stearic acid by transesterification.

Lipase-catalyzed transesterification reactions are reversible, and while the reaction may be progressed by the use of excess of the transesterification agent providing the additional fatty acid residues, these agents may be expensive and may entail further expense in their recovery and further refining for re-use.

The present invention proposes the conversion in such transesterification reactions of triglycerides containing residues of short-chain fatty acids in the presence of a lipase transesterification catalyst and a long-chain fatty acid or alkyl ester thereof and removing short-chain fatty acids or their alkyl esters from the reaction product to progress the transesterification reaction towards completion.

By promoting progress towards completion of the transesterification reaction in the process of the invention, the product may be recovered substantially free from incompletely substituted by-products and unreacted components without the necessity for their separation by fractional crystallization from the desired product and without the application of a gross excess of the transesterification reactant. Where a di-substituted product is required, for example by the use of 1-, 3-selective enzymes, substantially no mono-substituted by-products are formed, with only a moderate excess of the acid (or alkyl ester) reactant.

In general, the boiling point of fatty acids and their alkyl esters is determined by their carbon number, the presence of unsaturation having little effect. Preferably, the short-chain acid contains from 2 to 10 carbon atoms, more particularly 4 carbon atoms. Alkyl esters of such acids are preferably the methyl or ethyl esters.

When the process of the invention is applied to mixtures of triglycerides containing both short- and long-chain fatty acid residues, transesterificztion is promoted towards completion in accordance with the invention only in respect of those positions in which the catalyst is active and occupied by short-chain fatty acids. A non-selective catalyst will therefore promote complete replacement of short-chain acids in any of the glyceride positions in the process of the invention. A catalyst which is regio-specific, for example in the 1- and 3-positions of glycerides, will promote the replacement of short-chain fatty acids in these positions in accordance with the present invention.

It will be understood that in the process of the present invention all the fatty acid residues of the reactant triglycerides in those positions in which the catalyst is active, may be susceptible to replacement but that the replacement will be more complete in respect of the short-chain fatty acid residues.

The fat or glyceride oil containing short-chain fatty acid residues may be obtained from natural sources or may be prepared by synthesis or by a preliminary enzyme rearrangement process. Important examples of naturally-occurring fats include butterfat and lauric fats, e.g. coconut oil, palmkernel oil, babassu oil, seed fats of **Euonymus** and **Cuphea llavea (Lythrarieae)**, and their fractions obtained by fractional crystallisation. These examples contain a substantial proportion of their short chain fatty acid residues in the sn-3-position of the triglycerides of which they are principally composed their 2-positions principally containing long-chain fatty acid residues. Thus, more than 40% of the fatty acids in the sn-3-position of butterfat consists of butyric and caproic acids, only 0.1% of the fatty acids in the 2-position consisting of these acids, which amount in total to approximately 14% of the total fatty acid composition of butterfat. By the application of the present invention to butterfat therefore, substantially all these short-chain fatty acids ray be displaced by non-volatile, long-chain fatty acids, either saturated or unsaturated according to requirements. Thus, a long-chain fatty acid, whether saturated or unsaturated, and particularly polyunsaturated fatty acid, may be incorporated into butterfat by the present invention, to provide a modified butterfat composition, the properties of which are attributable in part to the newly acquired fatty acid residues. For this purpose, the use of a 1-, 3-selective catalyst is preferred. The present invention therefore provides, e.g., a modified butterfat substantially free from short-chain fatty acids, in which long-chain fatty acid residues, i.e. $C_{12}$ upward, have replaced the former short-chain fatty acid residues. Suitable examples of long-chain polyunsaturated fatty acids include in particular those of significance in human nutrition, e.g. the omega-3 and omega-6 $C_{20}$ and $C_{22}$ acids, e.g. pentaenoic including arachidonic and hexaenoic acids and the alpha and gamma linolenic acids, and in particular eicosapentaenoic and docosahexaenoic acid.

The application of the invention to lauric fats and their fractions also leads to valuable products. The bulk of the short-chain fatty acid residues containing not more than 8 carbon atoms are contained in the 1-, 3-positions of these fats. They may therefore be replaced in accordance with the present invention by saturated or unsaturated fatty acids of longer chain length, according to the requirements of the end-product. The well-known tendency of these fats to develop a soapy taste on storage is attributable, at least in part, to enzymatic hydrolysis liberating these shorter-chain fatty acids on storage. Their replacement by longer-chain fatty acids in accordance with the invention minimises this effect and may also confer additional benefits to the fat, according to the nature and characteristics of the replacement acids. The invention therefore also provides modified lauric fats substantially free from short-chain fatty acids, e.g. of less than $C_{12}$ chain length. Similarly, the invention provides substituted fats from the **euonymus** species, the acetic acid residues of which are substantially completely replaced by long-chain fatty acid residues, and, correspondingly, substituted fats of the **cuphea** species.

The replacement of short-chain fatty acid residues by long-chain saturated fatty acid residues in e.g. lauric fats and butterfat also provides valuable hardstock fats for the preparation of margarine and like spreads and of creams and emulsion products generally, whether for food or other applications.

An important advantage of the present invention is that long-chain unsaturated fatty acids have an important metabolic significance but are expensive to obtain either as free fatty acid or as short-chain alkyl esters but can be fully converted into triglycerides in the process of the invention without loss in unreacted form, by using only a moderate or no stoichiometric excess of the short-chain triglyceride reactant.

Triglycerides containing short-chain fatty acid residues may be prepared for use in the present invention from natural or other fats containing longer-chain fatty acid residues. These may be replaced, in part at least, in a preliminary enzyme transesterification process, using as transesterification agent a volatile short-chain fatty acid or its alkyl ester. The reversible enzymatic reaction may be promoted towards completion in this case by the use of a substantial excess of the short-chain fatty acid or its ester, for example methyl or ether esters of fatty acids containing from 1 to 4 carbon atoms, these fatty acids and their esters being relatively cheap.

The present invention therefore additionally provides an improved process for the transesterification of triglycerides under the influence of an active lipase enzyme as transesterification catalyst in which a triglyceride composition is reacted in successive stages with fatty reactants comprising short- and long-chain fatty acids or alkyl esters thereof, respectively, and evaporating volatile acid or ester from the desired product phase, at least in the final stage, to promote completion of the transesterification.

The fatty acid residues of the first stage preferably contain up to 4 carbon atoms, whereas those of the second stage of the process preferally contain at least 12 carbon atoms. In both stages the fatty acid moieties may be provided by free fatty acids or by esters, preferably alkyl esters, containing up to 4 carbon atoms in the alkyl group.

An important advantage of this two-stage process according to the invention is that the first stage may be promoted towards completion by using a substantial excess of cheap short-chain fatty acid or ester. The removal of the excess from the transesterified product of the first stage, if desired, is also facilitated by its high volatility and is preferably carried out at reduced pressure and moderately elevated temperature. Similarly, any solvent present may be evaporated from the product of either or both stages; the final product of the second stage may be crystallized from the liquid reaction phase. Alternatively, or in addition, fatty acids, or their alkyl esters, may also be evaporated from the product of the first stage, either before or during the second stage.

Evaporation of volatile acid or alkyl ester in either stage may take place in the same zone as the catalyst, where the temperature and/or pressure necessary to do so can be tolerated without adversely affecting the catalyst or in a separate zone from which the catalyst is excluded, the liquid phase being circulated continuously between the reaction zone to which the catalyst is confined and a phase transfer zone at which temperature and, if necessary, pressure are maintained to ensure the selective removal from the liquid phase of the volatile fatty acid or ester. By this mode of operation the reaction may be continued in the reaction zone to which the catalyst is confired, under moderately elevated temperature conditions at which the catalyst is effective and effectively isolated from the phase transfer zone, in which more extreme temperature and reduced pressure conditions can therefore be used without. affecting the catalyst, for evaporating the volatile fatty acid or ester.

The process of the present invention may be carried out batchwise or in continuous fashion and the two reaction stages may be conducted continuously in series in the same or separate reaction zones.

Preferably, the catalyst in each stage is supported on a suitable inert support and packed in a column through which the reactants are passed. Either or both stages may be carried out in the presence of a suitable water-immiscible solvent. The lipase enzyme may be non-selective or selective in regard to its reactivity towards specific fatty acids or specific stereo positions, particularly 1-, 3-selective in regard to glycerides. The invention is particularly applicable to the preparation of glycerides for pharmaceutical and edible purposes, including special fats for infant food formulations.

The proportions of triglycerides to free fatty acid or alkyl ester in the first stage is preferably at most 1:6 by weight, more particularly 1:10 by weight, to ensure a high degree of conversion, particularly using 1-, 3-selective catalyst.

The process of the invention may be conducted with advantage by recycling product acids or their alkyl esters, or by-products, for example partial glycerides separated from the rearranged triglycerides, if necessary after hydrolysis and/or hydrogenation. Thus, an unsaturated first-stage, long-chain product acid or ester thereof may be hydrogenated and recycled to the second state. Vice versa, the short-chain, second-stage product acid or ester thereof may be recycled to the first stage. This is particularly useful in the conversion of natural, unsaturated triglycerides from animal, marine or vegetable sources to more saturated homologues, for example in their conversion using 1-, 3-selective enzyme fats suitable for confectionery and containing symmetrical disaturated triglycerides of $C_{16}$ and $C_{18}$ fatty acids.

## EXAMPLE

A liquid fat mixture synthesized via enzymatic interesterification comprising :
45 wt.% of Bu P Bu;

43 wt.% of BU P P;
7 wt.% of PPP;
5 wt.% of other components containing Bu, P, St and O,
was mixed with ethyl oleate that was three times its weight. The mixture obtained was treated batchwise with a 1.3-specific lipase ( Mucor Miehei , NOVO) on a support (Duolite $^R$ ) for 2 hours at a temperature of 60 ° C. The water content of the total system was minimal, because all the ingredients had been dried before use.

The reaction was carried out in two different ways :

1) the pressure during the reaction was atmospheric so that no short-chain ethyl esters were removed;

2) the pressure during the reaction was about 20 mm so that the short-chain ethyl esters were removed. A rotary evaporator was used for this stage.

The crude reaction products of both procedures were worked up by filtration to remove the supported enzyme, followed by molecular distillation (T = 125 ° C, P = 0.05 mm).

In this way, reaction products were obtained, which contained 20% OPO (procedure 1) and 60% OPO (procedure 2).

The reaction product of procedure 2 did not contain any buteric acid moiety; only OPO, PPP and OPP were present.

## Claims

1. A modified lauric or butterfat composition with short-chain fatty acid residues and long-chain fatty acid residues, which is obtainable by replacement of short-chain fatty acid residues present in a non-modified lauric or butterfat.

2. Composition according to Claim 1, in which the long-chain fatty acid residues include a pharmalogically active acid.

3. Composition according to Claim 2, in which the said acid comprises eicosapentaenoic or docosahexaenoic acid.

4. Process for the transesterification of fats and triglyceride oils containing short-chain fatty acid residues, in the presence of a lipase transesterification catalyst and a long-chain fatty or alkyl ester thereof in which short-chain fatty acids or alkyl esters thereof are removed from the transesterification product to progess the transesterification reaction towards completion.

5. Process according to Claim 4, in which the short-chain fatty acids or esters are removed by evaporation under reduced pressure.

6. A modification of the process according to Claim 4 or 5, in which the starting, short-chain, fatty acid-containing triglyceride product is obtained by reaction of a fat or triglyceride oil with a short-chain fatty acid or alkyl ester thereof by transesterification under the influence of a lipase enzyme transesterification catalyst.

7. Process according to Claim 6, in which the short-chain fatty acid or ester is present in substantial excess to progress the transesterification reaction towards completion.

8. Process according to Claims 4 to 7, in which each product is recovered by evaporation of acid and/or alkyl ester.

9. Process according to Claim 8, in which fatty acid and/or ester removed by evaporation is saturated by hydrogenation where necessary and recycled.

10. Process according to Claims 4 to 9, in which transesterification is conducted ir the presence of a volatile, water-immiscible, organic solvent.

11. Process according to Claims 4 to 10, in which the catalyst is supported on a carrier and packed in a column through which the reactants are passed.

12. Process according to any of the preceding Claims, in which the catalyst is 1-, 3-selective.